# EUROPEAN PATENT SPECIFICATION

(11) **EP 3 930 643 B1**
(45) Date of publication and mention of the grant of the patent: **11.09.2024**
(21) Application number: 20708577.0
(22) Date of filing: 21.02.2020
(51) Int. Cl.: A61F 5/445

(54) **A VALVE FOR A UROSTOMY APPLIANCE**
VENTIL FÜR EIN UROSTOMIEGERÄT
VALVE POUR APPAREIL D'UROSTOMIE

(30) Priority: 28.02.2019 GB 201902746
(43) Date of publication of application: 05.01.2022
(73) Proprietor: Salts Healthcare Limited, West Midlands B7 4AA (GB)
(72) Inventor: ALLEN, Marcus, Birmingham West Midlands B7 4AA (GB)
(74) Representative: Forresters IP LLP
(86) International application number: PCT/GB2020/050414
(87) International publication number: WO 2020/174218

(56) References cited:
- WO-A1-2009/012304
- US-A- 4 306 705
- US-A1- 2015 190 272

## Description

### Description of Invention

Embodiments of the present invention relates to a valve for a urostomy appliance.

Urostomy appliances are well known in the field. They are typically attached to a patient via an adhesive wafer that extends around the patient's stoma with adhesive and provide a collecting chamber to collect waste (mostly liquid waste) exiting the stoma. A mechanism for draining the collecting chamber is often provided - typically, these are in the form of a tap or bung, which allow the patient to open an outlet from the urostomy appliance and drain the contents, for example, into a toilet.

WO2009/012304 discloses a urine drainage bag including a tap. The tap includes a tap member mounted to the housing so as to be pivotably movable between a closed, stowed position and an open, deployed position.

The current invention aims to alleviate one or more problems associated with to prior art urostomy appliance valves.

According to a first aspect of the present invention we provide a valve for a urostomy appliance including: a body for connection to the urostomy appliance, an inlet and an outlet, connected by a flow path, and a blocking member supported by the body and generally linearly moveable between a closed position, in which the blocking member blocks the inlet, such that liquid cannot flow through the flow path to the outlet, and an open position in which the inlet is open, such that liquid is permitted to flow through the flow path to the outlet, and a cover member which is moveable between an open position, in which liquid is permitted to flow through the outlet, and a closed position, in which liquid is prevented or inhibited from flowing through the outlet, and wherein the cover member is biased towards its open position, and wherein the cover member includes a holding formation and the blocking member includes a corresponding formation, such that when the holding formation engages the corresponding formation the cover member is held in its closed position.

Additional features of the present invention are outlined in the appended claims.
Figures 1 and 2 show a urostomy appliance in accordance with an embodiment of the present invention,
Figure 3 shows a valve in accordance with an embodiment of the present invention,
Figures 4 to 6 show various views of a valve in an open position in accordance with an embodiment of the invention,
Figure 7 shows a valve in accordance with an embodiment of the present invention,
Figure 8 shows a valve in accordance with an embodiment of the present invention,
Figure 9 shows a valve in accordance with an embodiment of the present invention,
Figure 10 shows an exploded view of a valve in accordance with an embodiment of the present invention,

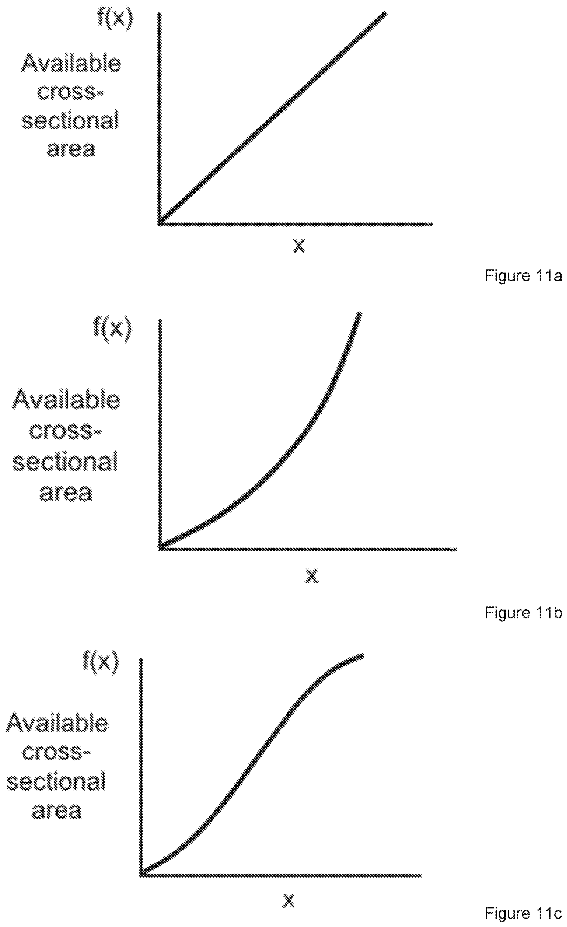

Figures 11a, b, c illustrate the available cross-sectional area of different inlet shapes, and
Figure 12 illustrates a connector for connecting to a valve.

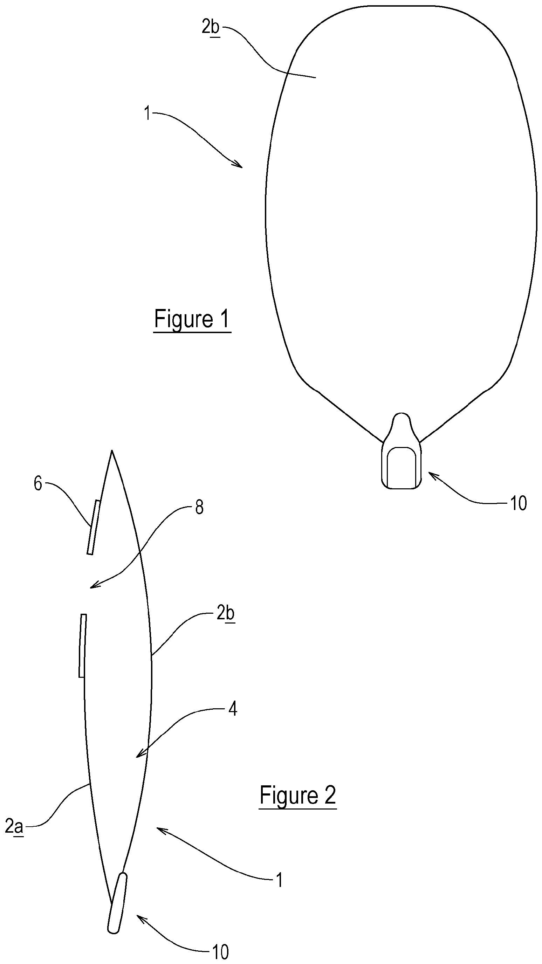

With reference to figures 1 and 2, particularly, a urostomy appliance 1 is illustrated. The urostomy appliance 1 includes first and second walls 2a, 2b which are connected together (for example, via a heat weld) to form a waste collecting cavity 4. The first wall 2a is attached to an adhesive wafer 6. An aperture (known as a stoma receiving opening 8) extends through both the first wall 2a and the adhesive wafer 6 to provide an entrance to the waste collecting cavity 4.

A valve 10 (described in more detail below) is attached to the second wall 2b of the urostomy appliance 1. The valve 10 communicates with the waste collecting cavity 4 and has an open and a closed position, in which waste is or is not permitted to flow through the valve 10 (i.e. flow out of the waste collecting cavity 4).

In use, the patient attaches the adhesive wafer 6 around their stoma. Waste liquid (for example, urine and/or blood and/or other body fluids) exits the body, via the stoma, and flows through the stoma receiving opening 8 and is collected in the waste collecting cavity 4. The valve 10 is selectively used to permit the waste which is collected in the waste collecting cavity 4 to flow out of the appliance 1, through the valve 10 (so that, for example, the appliance 1 can be emptied of some/most/all of the contents).

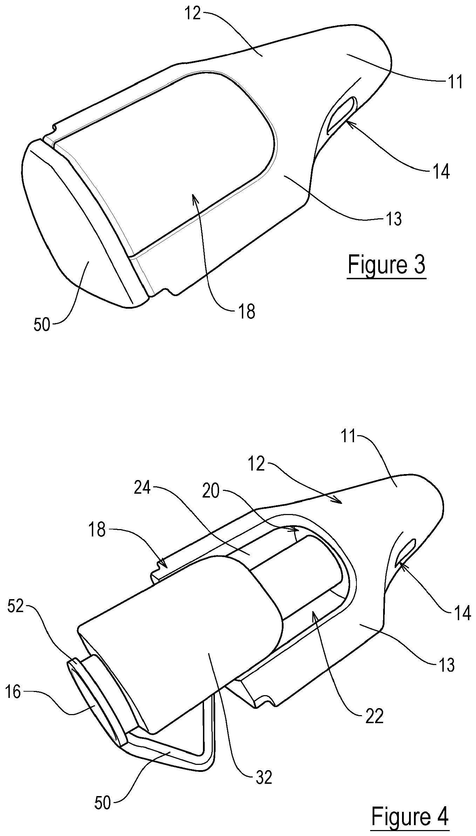

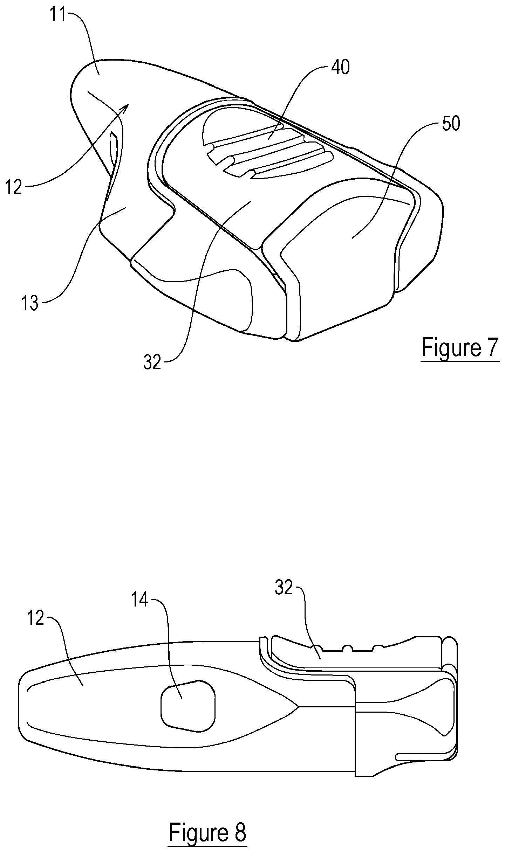

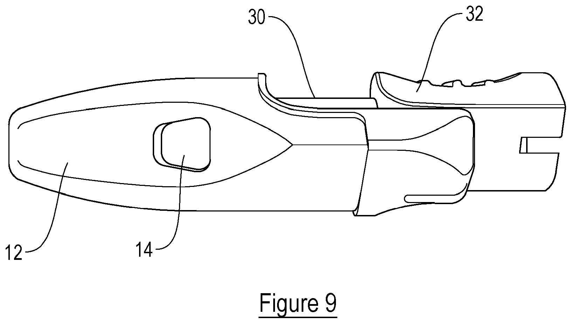

Features of the valve 10 are shown in more detail in figures 3 to 10. The valve 10 includes a body 12 for connection to the urostomy appliance 1, an inlet 14, and an outlet 16, which are connected by a flow path, and a blocking member 18, which is supported by the body 12.

In some embodiments (see particularly figures 3, 4, 7 and 10), the body 12 includes a tip that has a rounded and narrowed end 11 and a wider base part 13. In this case, the inlet 14 is defined by the body 12 (for example, the inlet 14 is formed adjacent the narrowed end of the body 12 and communicates with a body passage 20 that extends through part of the body 12. In some embodiments, the body 12 includes a further inlet (i.e. there are two inlets 14 in total). The further inlet is located on the opposing "side" of the body 12. In other words, the further inlet is positioned so that it also communicates with the body passage 20. In such a design, the further inlet is opened and closed synchronously with the inlet 14 (by the movement of the blocking member 18). It should be appreciated that two such inlets 12 are not necessarily required and there may be fewer or more as desired in a specific design.

In some embodiments, the inlet (or inlets) 14 are shaped in one of a tear-drop, egg/oval, trapezoidal, pentagonal or kite shape when viewed in a direction liquid passes therethrough.

In the illustrated embodiment, the body 12 also includes a channel 22. The channel 22 extends away from an entrance/exit of the body passage 20 and provides support for the blocking member 18 (the function of the channel 22 is described in more detail below).

The body 12 is connected to the urostomy appliance 1 by a heat weld (not shown) around the body 12, such that the inlet 14 communicates with the waste collecting cavity 4 (for example, the heat weld may extend across the base part 13 of the body 12, such that the narrowed end 11 and inlet 14 is in the urostomy appliance 1 / waste collecting cavity 4). It should be appreciated that the body 12 need not be heat welded necessarily. The body 12 may be attached to the appliance 1 in another manner that permits the inlet 14 to communicate with the waste collecting cavity 4.

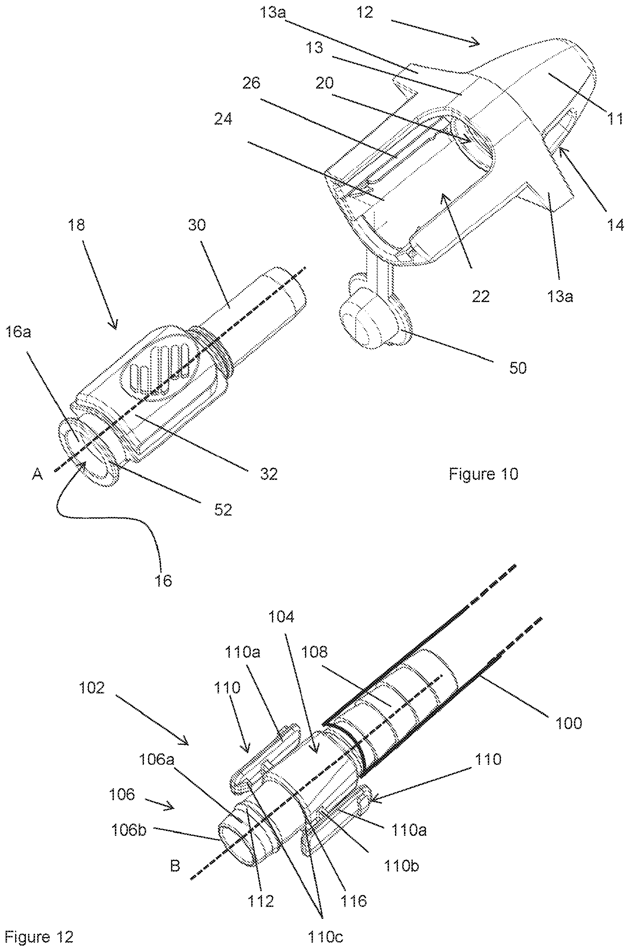

The embodiment illustrated in figure 10 shares the features of the valve 10 described in relation to the other figures (but only some features are labelled explicitly). In this case, the base part 13 of the body 12 includes two projections 13a which extend outwards from the sides of the base part 13. Each projection 13a includes an upper surface and a lower surface, which are inclined with respect to each other so that the edge furthest from the base part 13 is narrow (in the illustrated version, the upper and lower surfaces form a point at the edge furthest from the base part 13). The side projections 13a improve the manufacturing process by making the heat weld between the body 12 and urostomy appliance 1 easier to form because the films (or the urostomy appliance 1) that form the seal around the valve 10 (that seal around the entire base part 13) form a smoother, less extreme curve, around the projections 13a than if there were no projections present. It should be appreciated that any embodiment may include this feature, as desired.

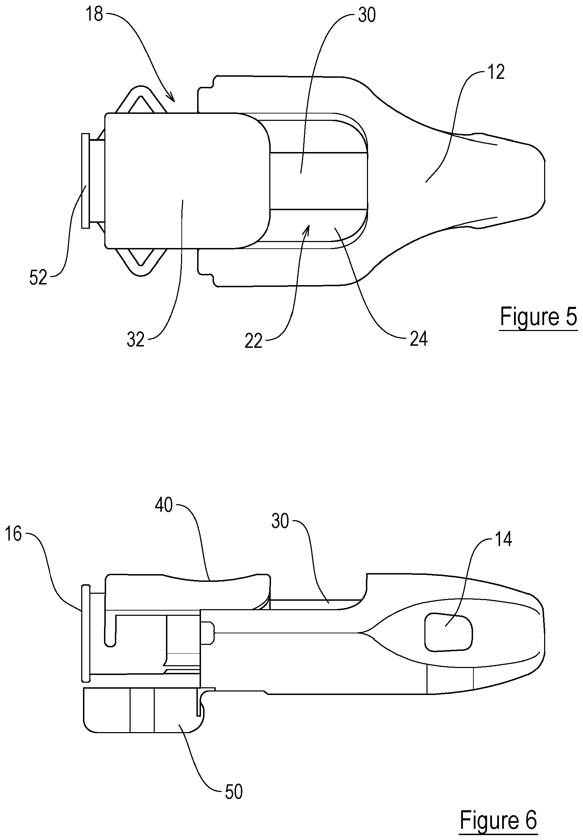

In some embodiments, a main part of the body 12 (i.e. the top and wider base part forming the body passage 20 and the channel 22) is made of a first material. The body 12 also includes a guide channel 24 (see figures 4 and 5), which is integrally formed (in this case, along a base of the channel 22). The guide channel 24 is made of a second material. The second material is more rigid than the first material.

The first material may be relatively pliable which means the body 12 is deformable and can be distorted reasonably easily. This allows the body 12 to be attached and sealed to the urostomy appliance 1 relatively easily but may make the body 12 more prone to damage if it is handled carelessly by a user (for example, if the valve 10 is squashed/distorted repeatedly). By including a second material to form a guide channel 24 that is more rigid than the rest of the body 12 may reduce distortion of the body 12 and may result in less damage being inflicted on the valve 10 during its useable life. It should be appreciated that this configuration may only be necessary if such the first material used for the body 12 is pliable / distortable. In some embodiments, the body 12 may be made from a first material that provides enough rigidity for the valve to operate as desired. It should also be appreciated that the first and second materials may be the same material but formed differently to provide different physical characteristics (e.g. two forms of PTFE).

It should be appreciated that the body 12 and the blocking member 18 may be manufactured using an overmoulding process. The blocking member 18 and/or the body 12 may be overmoulded with a rubber or rubber-like material.

The blocking member 18 is supported by the body 12 and is moveable between a closed position and an open position (as illustrated in figures 3 and 4, respectively). In the illustrated example, the blocking member 18 is generally linearly moveable between its closed open positions (and vice versa), but this does not necessarily have to be the case.

In the illustrated embodiments, the blocking member 18 includes a blocking portion 30 and a user operable part 32. A passage extends through the blocking member 18 (in this case, through both the blocking portion 30 and the user operable part 32) to connect to the outlet 16. In the present example, the opening extends substantially centrally, but this need not be the case.

When the valve 10 is assembled, and the blocking member 18 is in its closed position, the blocking portion 30 is received in the body passage 20 and the user operable portion 32 is received by the channel 22 (see figure 3). Thus, the passage through the blocking member 18 communicates with the body passage 20 through the body 12 to provide the flow path (between the inlet 14 and the outlet 16) through the valve 10 (whether the flow path is open for liquid to flow through depends on the position of the blocking member 18 in the body passage 20).

In some embodiments, the blocking member 18 is received and supported by the guide channel 24. The channel 24 prevents or at least inhibits movement of the blocking member 18 that is not generally linear with respect to the body 12. In some embodiments, the guide channel 24 inhibits non-coaxial movement of the blocking member 18 when the blocking member 18 is moved between its closed and open positions.

In some embodiments, the guide channel 24 includes a formation 26 (see figure 10) that engages a corresponding formation on the blocking member 18. Such a formation 26 and corresponding formation may provide additional guidance for the blocking member 18 as it is moved between its open and closed positions. It should be appreciated that such a formation and corresponding formation may not be present.

In some embodiments, the formation 26 of the guide channel includes one or more formations, each of which extends along the guide channel 24 in the direction of movement of the blocking member 18. The corresponding formation on the blocking member 18 includes at least one further formation. When the respective formations engage with each other (i.e. as the blocking member 18 moves), they inhibit any movement apart from generally linear movement of the blocking member 18. The formations could be axially extending projections/ridges and may be positioned on the base of the guide channel 24 or on each side of the guide channel 24, for example.

In some examples, the user operable portion 32 includes a depression 40 to aid user control. In some examples, the depression 40 also has a textured surface. These features aid user control by providing an area for a finger or thumb to be placed and provide the force required to slide open the valve 10.

In some embodiments, the valve 10 has an indicator that allows a user to feel when the valve 10 is in a fully open position. For example, the channel 22 may include an upward projection. When the blocking member 18 is moved to its open position (i.e. linearly outwards from the body 12) a part may pass over the projection and provide an indication that the blocking member 18 is in its fully open position. This allows the user to be confident that the valve 10 is fully open.

Likewise, another indicator could be used to inform the user when the valve 10 is in a closed position. This would allow a user to be confident that the valve 10 is closed and will not leak, for example.

In some embodiments, the valve 10 includes a cover member 50. The cover member 50 is moveable between an open position (see figure 6) and a closed position (see figure 3). When the cover member 50 is in its open positon, liquid is permitted to flow through the outlet 16, and when the cover member 50 is in its closed position, liquid is prevented or at least inhibited from flowing through the outlet 16.

In some embodiments, the cover member 50 pivots downwardly and towards a user wearing the urostomy appliance 1 when it is moved to its open positon when the cover member 50 is moved or moves to its open position. In other words, when the cover member 50 is in its open position it may be located between the user and the blocking member 18.

In some embodiments, the cover member 50 includes a holding formation (not shown) and the blocking member 18 includes a corresponding formation 52. When the holding formation engages the corresponding formation 52 the cover member 50 is held in its closed position (over the end of the blocking member 18 and sealing the outlet 16 in order to prevent or at least inhibit drips of liquid from exiting the outlet 16).

In some embodiments, the cover member 50 may be biased to its open position. In other words, once the holding formation and the corresponding formation 52 are disengaged from each other the cover member 50 may automatically move away from the outlet 16.

In some embodiments, the cover member 50 is moved due to movement of another part of the valve 10. In an example, as the blocking member 18 is moved towards its open position, the blocking member 18 effects movement of the cover member 50 to its open position. In such embodiments, the cover member 50 may have no biasing or may be biased towards a closed position (such that opening of the blocking member 18 controls the opening of the cover member 50).

The cover member 50 is attached to the body 12. In some embodiments, the cover member 50 includes a resiliently biased and flexible connection portion that extends to form the connection / attachment to the body 12.

Use of the valve 10 will now be discussed with reference to the features that have already been outlined above. In the closed position, the blocking member 18 blocks the inlet 14, such that fluid cannot flow through the valve 10. More specifically, when the valve 10 is in a closed position, the blocking portion 30 is positioned in the body passage 20 and closes off (i.e. seals) the inlet 14 (and liquid cannot flow through the inlet 14 and into the valve 10).

In the open position, the inlet 14 is open, such that fluid is permitted to pass through the valve 10. More specifically, when the valve 10 is in the open position, the blocking portion 30 is moved to an "outer" position, the inlet 14 communicates with the opening through the blocking member 18. In this configuration, liquid is permitted to exit the valve 10 via the outlet 16. It should be appreciated that such an "open" position refers to a fully open position in which the inlet 14 is open to its fullest extent (so the blocking portion 30 blocks the inlet 14 to the least extent possible) to allow a maximum flow rate of liquid to flow through the valve.

There is a range of partially open positions in which the blocking member 18 can be positioned which will result in a different flow rate of liquid being permitted to flow through the inlet 14 (due to it being partially open) and, thus, through the valve 10. In other words, the inlet 14 has a (total) cross-sectional area that is dictated by its size and shape. The inlet 14 also has an "available cross-sectional area" which is the available area (during use) through which liquid may flow. While the total cross-sectional area of the inlet 14 will not change substantially (it may change slightly through use of the valve 10 and general wear), the size of the available cross-sectional area depends on the position of the blocking member 18 (until the blocking member 18 reaches the fully open position and the entirety of the inlet 14 is open and available for liquid to flow through).

The inlet 14 is shaped such that as the blocking member is moved between a position in which a flow path is initially opened and a fully open position, the rate of change of the permitted flow rate of liquid permitted through the valve 10 increases with non-linear proportionality as the blocking member 18 is moved (although it should be appreciated that the rate of change of the permitted flow rate of the liquid through the inlet 14 may have both linearly proportional and non-linearly proportional portions). In other words, when considering the increase of the available cross-sectional area of the inlet 14 as the blocking member 18 is moved from initially open to fully open, the increase is non-linear.

For example, when the blocking member 18 is positioned at a distance *x* from its closed position, the available cross-sectional area is defined by a function (*f(x)).* When the blocking member 18 is positioned at a distance 2x from its closed position, the available cross-sectional area is defined by the same function (*f(2x)).* In this example, *f(2x)*> *2f(x)* (i.e. as the blocking member 18 is moved towards its open position from an initially open position, each unit of movement results in a small change in the available cross-sectional area of the inlet 14 and as the blocking member moves further the change in the available cross-sectional area becomes larger and larger until the fully open position).

In the present example, the inlet 14 is narrowed at one and becomes wider at an opposing end. The inlet 14 is oriented so that the narrower part becomes open first (as the blocking portion 30 moves outwards along the channel 20). As the blocking portion 30 moves further outwards, the wider parts of the inlet 14 become open. Thus, in this example the rate of change of the flow rate through the valve 10 will increase faster than it would for a rectangular or square inlet. In some embodiments, the inlet 14 has curved corners, which results in a different non-linear change in the flow rate.

The advantage of the inlet 14 being shaped in such a way that the flow rate of the liquid through the valve 10 is initially low and then increases more quickly (relative to a rectangular inlet 14, for example) is that a user can more easily control the direction of liquid flow when only a small amount of liquid is permitted to exit the valve 10. Once the user has established that the direction, etc. of the flow is acceptable they can continue to move the blocking member 18 towards its fully open position (which is reached more quickly due to the shape of the inlet 14). Thus, a valve 10 that has a low liquid flow rate initially (where the blocking member 18 is positioned at a distance *x*) and increases more quickly due to a wider part of the inlet 14 (where the blocking member is positioned at a distance that is greater than x) is an advantageous arrangement.

The functions that dictated the available cross-sectional area of different shaped inlets are illustrated generally in figures 10a, b and c. A rectangular shape is illustrated in 10a, and as can been seen the increase in available cross-sectional area is linearly proportional with the distance *x* that the blocking member 18 has travelled. A trapezoidal shape is illustrated in figure 10b. As can be seen the flow rate/available cross-sectional area is small to start with and the increase is steep as the distance *x* the blocking member 18 moves is increased. This results in a slow flow rate initially (to provide an aid for the user) and then approaching the largest flow rate fast as the blocking member 18 is moved further. Thus, the increase in flow rate is proportional to the distance *x* that the blocking member 18 has moved (but is not linearly proportional).

Figure 10c illustrates a circular shape, which results in a slow increase in the available cross-sectional area initially, then a steep increase as the widest part of the circle is available, followed by a slowing of the increase of the available cross-sectional area at the fully open position.

In the illustrated example, the blocking member 18 "opens" the flow path for liquid through linear movement with respect to the body 12. It should be appreciated that the desired alteration of the flow rate could be achieved using a valve that includes a portion that rotates between its closed position and its open position (and vice versa). The inlet may be positioned on the rotating part and the available cross-sectional area may be increased / decreased as the rotating part rotates. In this case the "distance" that the rotating portion travels through is an arc rather than linear.

Before the valve 10 is opened to allow waste to flow out of the urostomy appliance 1, the user may connect a conduit or tube 100 to the valve 10, so that the waste flowing out of the urostomy appliance 1 flows into another, connected, receptacle. The receptacle could be a night drainage bag, for example, so that the user does not have to get up during the night to empty their urostomy appliance 1 as it is connected to another (bigger) volume.

Thus, a drainage system is provided by the combination of the urostomy appliance 1 (described above), valve 10 and a connector 102 that is connectable to the conduit 100. Features of the valve 10 that permit the connector 102 to connect to the valve 10 are described below. However, it should be appreciated that another urostomy appliance/valve combination could connect to the connector 102 as long as the valve provided the features outlined here in relation to connecting to the connector 102.

The valve 10 includes the outlet 16 that forms a first fitting having a recess and an internal surface 16a. In some embodiments, the first fitting includes a hollow cylinder, which provides at least part of a flow path out of the valve 10 and into/through the connector 102.

In some embodiments, the first fitting has a circumferentially extending flange portion 52 at or near an entrance to its recess which provides an end face. The flange portion 52 extends generally perpendicularly to an axis A that extends longitudinally through the valve 10.

An embodiment of the connector 102 is illustrated in figure 12. The connector 102 is connected or connectable at a first end to a conduit 100 (the conduit 100 may be connected by way of a spigot or push fit fitting 108, for example). The connector 102 is connected or connectable at a second end to the valve 10. The second end provides a second fitting including a projection 106 having an external surface 106a. In other words, the connector 102 has a main body 104 that extends between the first and second ends.

In some embodiments, the second fitting includes a hollow cylinder, which, in use, provides at least part of a flow path through the connector 102 to the connected conduit 100. In some embodiments, the second fitting has a circumferentially extending shoulder 116 that is spaced from its distal end 106b. The shoulder 116 extends generally perpendicularly to an axis B that extends longitudinally through the connector 102 (although it should be appreciated that this need not be the case as the shoulder may be angled other than at 90 degrees, e.g. generally transversely, to axis B and still provide its function).

In some embodiments, the connector 102 includes two holding formations 110 (although it should be appreciated that there may be more or fewer holding formations 110, as desired). The holding formations 110 are positioned on opposing sides of the main body 104 to each other (in the case of more than two holding formations, they may be spaced around the main body and in the case of one holding formation, it may be positioned to one side). Each holding formation 110 includes an elongate portion 110a that is attached to the main body 104 by an extension portion 11 0b. The elongate portion 110a extends generally parallel to the axis B and the extension portion 110b extends generally perpendicular to the axis B (away from the main body 104). The extension portion 110a connects to the main body 104 further away from the connector's distal end 106b than the shoulder 116. The elongate portion 110a extends beyond the shoulder 116, towards the distal end 106b, and overlies the second fitting (and, when connected to the valve 10, overlies the flange portion 52). The extension portion 110b connects the elongate portion 110a to the main body 104 with a degree of flexibility so as to provide a pivoting action that allows the elongate portion 110a to move out of general parallel alignment with the main body 104 / axis B.

The elongate portion 110a includes a camming surface 110c. In the illustrated case, the camming surface 110c is positioned at one end of the elongate portion 110a, which is closest to the shoulder 116 (it should be appreciated that there are other arrangements that provide the same functionality). The camming surface 110c faces inwardly, towards the main body 104 / second fitting. This allows the camming surface 110c to engage the flange portion 52 during use (described in more detail below).

The holding formation 110 or at least a part of the holding formation 110 is resiliently biased, so that it may be moved and/or deflected and return to its original position. In the present embodiment, the extension portion 110b provides a pivoting action that allows the elongate portion 110a to deflect outwards. However, it should be appreciated that the holding formation 110 in general is made of a plastics material that is resilient and, therefore, the elongate portion 110a is also able to deform in itself as well as in combination with the extension portion 110b. Thus, it should be appreciated that the extension portion 110b does not necessarily need to provide such a pivoting action in order for the holding formation 110 to function (i.e. hold the connector 102 to the valve 10).

In some embodiments, the second fitting includes a seal 112, which is provided on, connected to or forms part of the external surface 106a. The seal 112 is located in a position between the shoulder 116 and the distal end. In this illustrated case, the seal 112 extends continuously and in an annular shape in a plane that is perpendicular to axis B (but this may not be the case). In some embodiments, the seal 112 is formed using an overmoulding process.

In use, the second fitting (of the connector 102) is received in the first fitting (of the valve 10) when the connector 102 is connected to the valve 10. When the connector 10 is connected to the valve 10, the shoulder 116 engages the end face of the female fitting. Advantageously, the holding formations 110 engage the flange portion 52 such that the connector 102 and the valve 10 are held together and disconnection is inhibited or substantially prevented.

As the connector 102 is moved towards a "connected position", the respective camming surfaces 110a of the holding formations 110 are (automatically) deflected outwards over the flange portion 52. It should be appreciated that if camming surfaces 110a are not provided then a latch formed from a projection maybe present and a user can manually deflect the holding formations 110 over the flange portion 52. Advantageously, if the holding formation 110 is resiliently biased, it moves back to its original position once the camming surfaces have moved past the flange portion 52.

Also when the connector 102 is connected to the valve 10, the seal 112 engages the internal surface 16a of the first fitting. Thus, liquid leakage around the first / second fittings is minimised / inhibited / substantially prevented.

To disconnect the connector 102 from the valve 10, the opposite end of the elongate portion 110a to the camming surface 110c is pressed towards the main body 104, so as to deflect the latch / camming surface 110c over the flange portion 52 (and the connector 102 can be disconnected from the outlet 16 of the valve 10).

It should be appreciated that the conduit 100 has a connector 102 attached at one or both ends for connection to the valve 10 and the night drainage bag, respectively.

## Claims

1. A valve (10) for a urostomy appliance (1) including:
a body (12) for connection to the urostomy appliance (1),
an inlet (14) and an outlet (16), connected by a flow path, and
a blocking member (18) supported by the body (12) and generally moveable between a closed position, in which the blocking member (18) blocks the inlet (14), such that liquid cannot flow through the flow path to the outlet (16), and an open position in which the inlet (14) is open, such that liquid is permitted to flow through the flow path to the outlet (16), and
a cover member (50) which is moveable between an open position, in which liquid is permitted to flow through the outlet (16), and a closed position, in which liquid is prevented or inhibited from flowing through the outlet (16), and wherein the cover member (50) is biased towards its open position, and
wherein the cover member (50) includes a holding formation and the blocking member (18) includes a corresponding formation (52), such that when the holding formation engages the corresponding formation (52) the cover member (50) is held in its closed position **characterised in that** the blocking member is generally linearly moveable between the open position and closed position.

2. A valve (10) according to claim 1 wherein as the blocking member (18) is moved to its open position, the blocking member (18) effects movement of the cover member (50) to its open position

3. A valve (10) according to any of claims 1 or 2 wherein the cover member (50) includes a resiliently biased and flexible connection portion connecting the cover member (50) to the body (12).

4. A valve (10) according to any of the preceding claims wherein the cover member (50) is connected to the body (12) such that it pivots downwardly and towards a user wearing the urostomy appliance (1) when it is moved or moves to its open position.

5. A valve (10) according to claim 3 wherein as the blocking member (18) moves to its open position, the blocking member (18) moves in front of the cover member (50), such that the cover member (50) is positioned between the user and the blocking member (18).

6. A valve (10) according to any of the preceding claims wherein the body (12) has a body passage (20) that forms part of the flow path and communicates with a passage in the blocking member (18).

7. A valve (10) according to any of the preceding claims wherein the body (12) is formed from a first material and has an integrally formed guide channel (24) which is formed from a second material, said second material being more rigid than the first material, and
wherein the blocking member (18) is received in the guide channel (24) and the guide channel (24) is configured to prevent or inhibit movement of the blocking member (18) in any direction other than generally linearly as it moves from its closed position towards its open position.

8. A valve (10) according to claim 7 wherein the guide channel (24) includes a formation (26) which engages with a corresponding formation on the blocking member (18) to guide the blocking member (18) when moving between its open and closed positons.

9. A valve (10) according to claims 8 wherein the formation of the guide channel (24) includes one or more formations, each of which extends along the guide channel (24) in the direction of movement of the blocking member (18) and the corresponding formation on the blocking member (18) includes one or more corresponding formations which engage therewith as the blocking member (18) moves.

10. A combination of a urostomy appliance (1) and a valve (10), the urostomy appliance (1) including:
a first wall (2a) and a second wall (2b) connected about their respective peripheries to define an internal waste collecting cavity (4),
a stoma receiving opening (8) which is positioned in the first wall (2a) and is in communication with the waste collecting cavity (4), and
the valve (10) according to any of claims 1-9 connected to one of the first and second walls (2a, 2b).

## Patentansprüche

1. Ventil (10) für ein Urostomiegerät (1), beinhaltend:
einen Körper (12) zur Verbindung mit dem Urostomiegerät (1),
einen Einlass (14) und einen Auslass (16), die durch einen Durchflussweg verbunden sind, und
ein Blockierelement (18), das von dem Körper (12) getragen wird und im Allgemeinen zwischen einer geschlossenen Position, in der das Blockierelement (18) den Einlass (14) blockiert, so dass Flüssigkeit nicht durch den Durchflussweg zu dem Auslass (16) fließen kann, und einer offenen Position, in der der Einlass (14) offen ist, so dass es der Flüssigkeit gestattet ist, durch den Durchflussweg zu dem Auslass (16) zu fließen, bewegbar ist, und
ein Abdeckelement (50), das zwischen einer offenen Position, in der es der Flüssigkeit gestattet ist, durch den Auslass (16) zu fließen, und einer geschlossenen Position, in der verhindert oder inhibiert wird, dass Flüssigkeit durch den Auslass (16) fließt, bewegbar ist, und wobei das Abdeckelement (50) hin zu seiner offenen Position vorgespannt ist, und
wobei das Abdeckelement (50) eine Halteformation beinhaltet und das Blockierelement (18) eine entsprechende Formation (52) beinhaltet, so dass, wenn die Halteformation mit der entsprechenden Formation (52) in Eingriff steht, das Abdeckelement (50) in seiner geschlossenen Position gehalten wird, **dadurch gekennzeichnet, dass** das Blockierelement im Allgemeinen linear zwischen der offenen Position und geschlossenen Position bewegbar ist.

2. Ventil (10) nach Anspruch 1, wobei, da das Blockierelement (18) zu seiner offenen Position bewegt wird, das Blockierelement (18) eine Bewegung des Abdeckelements (50) zu seiner offenen Position bewirkt.

3. Ventil (10) nach einem der Ansprüche 1 oder 2, wobei das Abdeckelement (50) einen elastisch vorgespannten und flexiblen Verbindungsabschnitt beinhaltet, der das Abdeckelement (50) mit dem Körper (12) verbindet.

4. Ventil (10) nach einem der vorhergehenden Ansprüche, wobei das Abdeckelement (50) mit dem Körper (12) verbunden ist, so dass es nach unten und hin zu einem das Urostomiegerät (1) tragenden Benutzer schwenkt, wenn es bewegt wird oder sich zu seiner offenen Position bewegt.

5. Ventil (10) nach Anspruch 3, wobei, da sich das Blockierelement (18) zu seiner offenen Position bewegt, sich das Blockierelement (18) vor das Abdeckelement (50) bewegt, so dass das Abdeckelement (50) zwischen dem Benutzer und dem Blockierelement (18) positioniert ist.

6. Ventil (10) nach einem der vorhergehenden Ansprüche, wobei der Körper (12) einen Körperdurchgang (20) aufweist, der einen Teil des Durchflusswegs bildet und mit einem Durchgang in dem Blockierelement (18) kommuniziert.

7. Ventil (10) nach einem der vorhergehenden Ansprüche, wobei der Körper (12) aus einem ersten Material gebildet ist und einen integral geformten Führungskanal (24) aufweist, der aus einem zweiten Material gebildet ist, wobei das zweite Material starrer als das erste Material ist, und
wobei das Blockierelement (18) in dem Führungskanal (24) aufgenommen wird und der Führungskanal (24) dazu konfiguriert ist, eine Bewegung des Blockierelements (18) in einer anderen Richtung als allgemein linear zu verhindern oder zu inhibieren, da es sich von seiner geschlossenen Position hin zu seiner offenen Position bewegt.

8. Ventil (10) nach Anspruch 7, wobei der Führungskanal (24) eine Formation (26) beinhaltet, die mit einer entsprechenden Formation auf dem Blockierelement (18) in Eingriff steht, um das Blockierelement (18) bei einer Bewegung zwischen seiner offenen und geschlossenen Position zu führen.

9. Ventil (10) nach Anspruch 8, wobei die Formation des Führungskanals (24) eine oder mehrere Formationen beinhaltet, von denen sich jede entlang des Führungskanals (24) in der Bewegungsrichtung des Blockierelements (18) erstreckt, und die entsprechende Formation auf dem Blockierelement (18) eine oder mehrere entsprechende Formationen beinhaltet, die damit in Eingriff stehen, da sich das Blockierelement (18) bewegt.

10. Kombination aus einem Urostomiegerät (1) und einem Ventil (10), wobei das Urostomiegerät (1) beinhaltet:
eine erste Wand (2a) und eine zweite Wand (2b), die um ihre jeweiligen Peripherien verbunden sind, um einen inneren Fäkaliensammelhohlraum (4) zu definieren,
eine Stomaaufnahmeöffnung (8), die in der ersten Wand (2a) positioniert ist und in Kommunikation mit dem Fäkaliensammelhohlraum (4) steht, und
das Ventil (10) nach einem der Ansprüche 1-9, das mit einer der ersten und zweiten Wand (2a, 2b) verbunden ist.

## Revendications

1. Valve (10) pour un appareil d'urostomie (1), comprenant :
un corps (12) destiné à être raccordé à l'appareil d'urostomie (1),
un orifice d'entrée (14) et un orifice de sortie (16), reliés par un trajet d'écoulement, et un élément de blocage (18) que supporte le corps (12) et qui étant mobile peut généralement passer d'une position fermée, dans laquelle l'élément de blocage (18) bloque l'orifice d'entrée (14) de sorte que du liquide ne puisse pas s'écouler à travers le trajet d'écoulement jusqu'à l'orifice de sortie (16), à une position ouverte, dans laquelle l'orifice d'entrée (14) est ouvert, de sorte que du liquide puisse s'écouler à travers le trajet d'écoulement jusqu'à l'orifice de sortie (16), et
un élément capuchon (50) qui étant mobile peut passer d'une position ouverte, dans laquelle du liquide peut s'écouler à travers l'orifice de sortie (16), à une position fermée, dans laquelle l'écoulement du liquide à travers l'orifice de sortie (16) est entravé ou inhibé, et dans lequel l'élément capuchon (50) est sollicité vers sa position ouverte, et
dans lequel l'élément capuchon (50) comprend une structure de retenue et l'élément de blocage (18) comprend une structure correspondante (52), de sorte que lorsque la structure de retenue entre en prise avec la structure correspondante (52) l'élément capuchon (50) est maintenu dans sa position fermée, **caractérisé en ce que** l'élément de blocage peut généralement linéairement passer d'une position ouverte à une position fermée.

2. Valve (10) selon la revendication 1, dans lequel alors que l'élément de blocage (18) passe sur sa position ouverte, l'élément de blocage (18) provoque le déplacement de l'élément capuchon (50) sur sa position ouverte.

3. Valve (10) selon soit la revendication 1, soit la revendication 2, dans lequel l'élément capuchon (50) comprend une partie de raccordement sollicitée de manière élastique et souple reliant l'élément capuchon (50) au corps (12).

4. Valve (10) selon l'une quelconque des revendications précédentes, dans lequel l'élément capuchon (50) est relié au corps (12), de sorte qu'il pivote vers le bas et vers un utilisateur porteur d'un appareil d'urostomie (1) lorsqu'il est déplacé ou se déplace sur sa position ouverte.

5. Valve (10) selon la revendication 3, dans lequel, alors que l'élément de blocage (18) se déplace sur sa position ouverte, l'élément de blocage (18) se déplace devant l'élément capuchon (50), de sorte que l'élément capuchon (50) soit positionné entre l'utilisateur et l'élément de blocage (18).

6. Valve (10) selon l'une quelconque des revendications précédentes, dans lequel le corps (12) comporte un passage de corps (20) qui fait partie du trajet d'écoulement et qui communique avec un passage prévu dans l'élément de blocage (18).

7. Valve (10) selon l'une quelconque des revendications précédentes, dans lequel le corps (12) est formé à partir d'une première matière et comporte un canal de guidage formé d'un seul tenant (24) qui est formé à partir d'une seconde matière, ladite seconde matière étant plus rigide que la première matière, et
dans lequel l'élément de blocage (18) vient se loger dans le canal de guidage (24) et le canal de guidage (24) est configuré de sorte à entraver ou à inhiber le déplacement de l'élément de blocage (18) dans toute direction autre qu'une direction généralement linéaire alors qu'il passe de sa positon fermée vers sa position ouverte.

8. Valve (10) selon la revendication 7, dans lequel le canal de guidage (24) comprend une structure (26) qui entre en prise avec une structure correspondante sur l'élément de blocage (18) pour guider l'élément de blocage (18) lors de son passage de sa position ouverte à sa position fermée.

9. Valve (10) selon la revendication 8, dans lequel la structure du canal de guidage (24) comporte une ou plusieurs structures, chacune d'entre elles s'étendant le long du canal de guidage (24) dans la direction du déplacement de l'élément de blocage (18), et la structure correspondante sur l'élément de blocage (18) comporte une ou plusieurs structures correspondantes qui se entrent en prise avec celle-ci alors que se déplace l'élément de blocage (18).

10. Association d'un appareil d'urostomie (1) et d'une valve (10), l'appareil d'urostomie (1) comprenant :
une première paroi (2a) et une seconde paroi (2b) jointes de part et d'autre de leurs pourtours respectifs pour définir une cavité de collecte de déchets internes (4),
une ouverture de recueil de stomie (8) qui est située dans la première paroi (2a) et qui est en communication avec la cavité de recueil de déchets (4), et
la valve (10) selon l'une quelconque des revendications 1 à 9, qui est reliée à l'une ou l'autre de la première et de la seconde parois (2a, 2b).
